# EUROPÄISCHE PATENTSCHRIFT

(11) **EP 4 304 674 B1**
(45) Veröffentlichungstag und Bekanntmachung des Hinweises auf die Patenterteilung: **23.09.2026**
(21) Anmeldenummer: 22715021.6
(22) Anmeldetag: 14.03.2022
(51) Int. Cl.: A61M 1/16

(54) **MISCHVORRICHTUNG ZUR HERSTELLUNG VON DIALYSELÖSUNGEN**
MIXING DEVICE FOR THE PRODUCTION OF DIALYSIS SOLUTIONS
DISPOSITIF MÉLANGEUR POUR LA PRÉPARATION DE SOLUTIONS POUR LA DIALYSE

(30) Priorität: 13.03.2021 DE 102021001350
(43) Veröffentlichungstag der Anmeldung: 17.01.2024
(73) Patentinhaber: Lohse, Marvin, 33739 Bielefeld (DE)
(72) Erfinder: WESSELER, Matthias, 49326 Melle (DE); LOHSE, Marvin, 33739 Bielefeld (DE)
(74) Vertreter: Hoffmann Eitle
(86) Internationale Anmeldenummer: PCT/EP2022/056575
(87) Internationale Veröffentlichungsnummer: WO 2022/194796

(56) Entgegenhaltungen:
- DE-A1- 102013 102 914
- US-A1- 2019 262 521

## Beschreibung

Die vorliegende Erfindung betrifft eine Vorrichtung zur Zubereitung von Dialyse- oder Infusionslösungen, mit der die Bereitstellung individualisierte Zusammensetzungen möglich ist. Die Vorrichtung soll sicherstellen, dass die Konzentration einzelner Stoffe nicht ungewollt unter- oder überschritten werden.

Es existieren verschiedene Verfahren zur Blutbehandlung eines Patienten, insbesondere für die Elimination von Stoffen aus dem Blut eines Patienten und zur Entwässerung des Blutes eines Patienten. Diese Verfahren werden Dialyseverfahren genannt. Ein weiteres Verfahren zum Ausgleich von zu niedrigem Blutvolumen ist die Infusion einer Lösung in die Blutbahn des Patienten.

Bei der Hämodialyse werden aus Blut des Patienten in einem extrakorporalen Blutkreislauf überschüssige Stoffe und Wasser eliminiert. Im extrakorporalen Blutkreislauf befindet sich typischrweise ein Dialysefilter, der zwei durch eine semipermeable Membran voneinander getrennten Kammern umfasst. Durch eine der Kammern wird das Blut des Patienten geleitet, durch die andere Kammer wird eine Spülflüssigkeit (Dialysat) geleitet. Aufgrund eines Stoffkonzentrationsgradienten zwischen dem Patientenblut und der Spülflüssigkeit werden durch den physikalischen Effekt der Diffusion und Osmose Substanzen und Wasser durch die semipermeable Membran hindurch transportiert.

Bei der Hämofiltration wird wird das Blut des Patienten in einen zur Hämodialyse ähnlichen extrakorporalen Blutkreislauf geleitet. Im Dialysefilter wird der Stofftransport durch die semipermeablen Membran hindurch durch einen Druckgradienten, also konvektiv, bedingt. Die durch den Druckgradienten abgeführte Flüssigkeit muss entweder vor oder nach dem Dialysefilter durch die Infusion von Substitutionslösung in den Blutkreislauf substituiert werden.

Die Kombination der Verfahren Hämodialyse und Hämofiltration wird Hämodiafiltration genannt.

Die Spülflüssigkeit und die Substitutionslösung können entweder als gebrauchsfähige Lösung oder als Konzentrat an eine Blutbehandlungseinheit angeschlossen werden. Wenn ein Konzentrat verwendet wird, wird dieses in der Blutbehandlungseinheit in einem definierten Verhältnis mit Wasser zu einer gebrauchsfertigen Lösung verdünnt.

Um die Ausfällung schwerlöslicher Stoffe bei der Auslieferung zu verhindern, wird die Spülflüssigkeit bzw. die Substitutionslösung zumeist in zwei oder mehr verschiedene Bestandteile aufgeteilt ausgeliefert und erst in der Blutbehandlungseinheit zur gebrauchsfertigen Lösung vermischt. Diese Bestandteile werden im Folgenden exemplarisch Bestandteil A und Bestandteil B genannt, es können aber auch, je nach Anbieter der Dialysebehandlungen, noch mehr Bestandteile verwendet werden. Spülflüssigkeit kann von der Blutbehandlungseinheit durch Sterilfiltration zu Substitutionslösung aufbereitet werden.

Das Vermischen und Verdünnen der Bestandteile A und B machen sich moderne Blutbehandlungseinheiten zunutze, indem sie durch eine geringere oder höhere Verdünnung von Bestandteil A und B ein Konzentrationsprofil über die Behandlungszeit erzeugen können. Verdünnt man beispielsweise den Bestandteil A weniger, so befindet sich in der Spülflüssigkeit eine höhere Konzentration aller in A enthaltenen Stoffe. Das relative Verhältnis der Stoffkomponenten von A zueinander verändert sich dadurch jedoch nicht, das relative Verhältnis der Stoffkomponenten aus A zu den Stoffkomponenten aus B jedoch schon.

Bei einer Dialysebehandlung nach dem Stand der Technik besteht einer der Bestandteile zu ca. 95% aus Natriumchlorid, der andere Bestandteil besteht vollständig aus Natriumbicarbonat. Durch die Veränderung der Verdünnungsverhältnisse lassen sich daher die Konzentration von Natriumchlorid oder Natriumbicarbonat in der fertigen Spülflüssigkeit variieren, während die Zusammensetzung der zusätzlichen 5% der in der Natriumchloridlösung vorhandenen Stoffe nur eine sehr geringe quantitativ absolute Änderung erfährt.

Da durch Sterilfiltration mittels Sterilfiltern die Spülflüssigkeit zu Substitutionslösung aufbereitet werden kann, gilt dieses Prinzip gleichermaßen auch für die Substitutionslösung. Ein vollständig voneinander unabhängiges Variieren sämtlicher Stoffe in Spülflüssigkeit oder in Substitutionslösung wird nach Stand der Technik nicht angewendet.

Eine weiterhin verbesserte Möglichkeit zur Anpassung der Stoffzusammensetzung der Spüllösung bzw. der Substitutionslösung ist anzustreben. Dies insbesondere, um damit einem Patienten eine individualisierte Behandlung zu ermöglichen, z.B. eine Anpassung der Lösung an seine individuelle Physiologie und sein individuelles Krankheitsbild. Außerdem ändert sich während einer Dialysebehandlung der Stoffhaushalt des Patienten im Intrasowie Extrazellulärraum. Dies wird bei der Dialysetherapie gemäß dem Stand der Technik nicht zufriedenstellend berücksichtigt.

Gemäß dem Stand der Technik wird die Therapieanpassung soweit realisiert, dass verschiedene konzentrierte oder gebrauchsfertige Lösungen mit verschiedenen Rezepturen vorliegen und in den Dialysestationen gelagert werden müssen. Für jeden Patienten muss dann zuvor eine passende Rezeptur (es existieren >50 verschiedene Rezepturen verschiedener Verdünnungsverhältnisse am Markt) ausgewählt werden und diese dann in Form des Konzentrates oder in gebrauchsfertiger Form an die Blutbehandlungseinheit konnektiert werden. Dies führt zu großem logistischen Aufwand, da vor und nach jeder Dialysebehandlung eine andere Rezeptur an die Blutbehandlungseinheit angeschlossen werden muss.

Logistisch günstiger wäre die Versorgung der Blutbehandlungseinheiten über eine zentrale Versorgung, bei der ein sehr großes Reservoir über Flüssigkeitsleitungen mit den Blutbehandlungseinheiten verbunden wird. Dabei werden die Patienten jedoch alle mit der gleichen Zusammensetzung der Spülflüssigkeit bzw. Substitutionslösung behandelt. Eine Individualisierung wäre somit nicht möglich.

Es sind bereits Vorrichtungen bekannt, die daran ansetzen, die Spülflüssigkeit oder Substitutionslösung an der Blutbehandlugseinheit zu produzieren, so z.B. aus der DE102013102914 A1. Bei dieser Vorrichtung werden die verschiedenen Bestandteile (zumeist Salze oder deren Lösungen) von voneinander getrennten Vorratsbehältern aus direkt in die Reinwasserleitung gefördert, und über diesen Weg wird die Mischung der Spülflüssigkeit erzeugt. Liegen die Salze in den Behältern in Reinform vor, benötigt man entsprechend eine Anzahl *nᵢ* = *nₛ* Fluideinlässe für eine unabhängig zu variierende Anzahl *nₛ* an Stoffen. Dieser Weg ist jedoch im Sinne der Patientensicherheit als kritisch zu bewerten, da bei einer Fehlfunktion der Mischvorrichtung zulassungsrechtliche oder therapeutisch notwendige Grenzen über- oder unterschritten werden könnten. Befindet sich beispielsweise in einem der Behälter hochkonzentriertes Kaliumchlorid, könnte der Patient mit einer zu hohen Dosis davon behandelt werden, was im schlimmsten Fall den Tod des Patienten zur Folge hätte.

Der Erfindung liegt somit die Aufgabe zugrunde, wenigstens einen der Nachteile aus dem Stand der Technik zu beheben oder wenigstens zu lindern.

Insbesondere ist es ein Vorteil der erfindungsgemäßen Vorrichtung, eine verbesserte Blutbehandlung zu ermöglichen, indem die Stoffzusammensetzung einzelner oder aller für die Behandlung des Patienten vorgesehenen Lösungen frei und unabhängig voneinander vor und/oder während der Behandlung variiert werden können, ohne dass dabei die vorgegebenen Grenzen in der Stoffkonzentration einzelner Stoffe in der für den Patentienten gebrauchsfertigen Lösung über- oder unterschritten werden können.

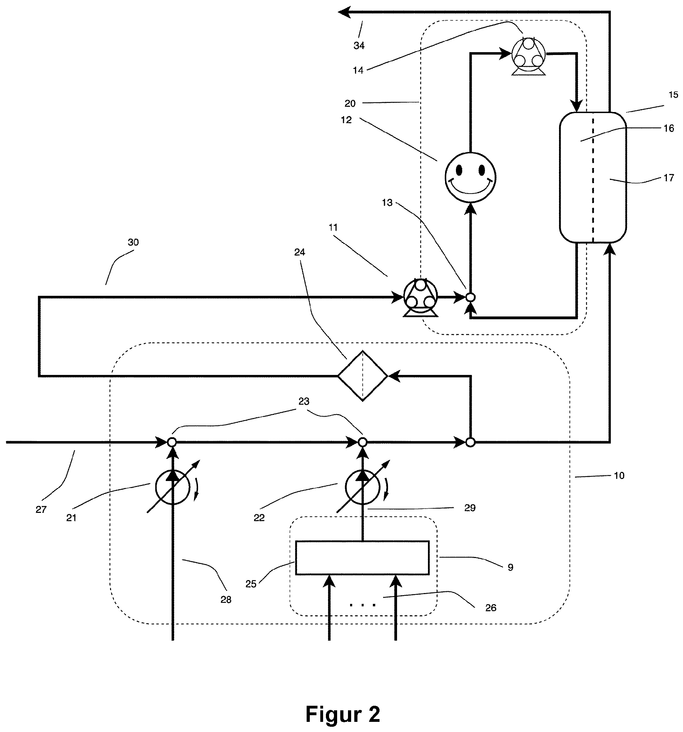

Dazu ist es vorteilhaft, dass die erfidnungsgemäße Vorrichtung der Dosiervorrichtung in der Blutbehandlungseinheit vorgeschaltet wird, an der bei einer Blutbehandlungseinheit nach dem Stand der Technik die saure Komponente (Komponente A) angeschlossen wird. Dabei kann die Mischvorrichtung je nach Ausführungsform entweder in die Blutbehandlungseinheit integriert sein (vgl. Fig. 2) oder sich als Zusatzmodul außerhalb der Blutbehandlungseinheit befinden. Dann wird dieses Zusatzmodul vorzugsweise über den Fluidauslass an die Blutbehandlungseinheit konnektiert.

Die technische Vorrichtung besitzt mindestens zwei Fluideinlässe für Basisrezepturen, wobei jeder Einlass jeweils mindestens eine Dosiervorrichtung (z.B. Pumpen, Ventile) besitzt. Durch diese Dosiervorrichtung stehen die Fluideinlässe in fluidischer Verbindung mit einem gemeinsamen Flüssigkeitsleitungssystem. Dieses Flüssigkeitsleitungssystem kann in einer bevorzugten Ausführungsform als Reservoir ausgestaltet sein oder ein Reservoir umfassen. Eine Regel- oder Steuerungseinrichtung kann mittels eines Sensors den physikalischen Zustand der Vorrichtung (z.B. Flussrate, Druck, Temperatur, Leitwert) monitoren. Die Dosiervorrichtung kann damit so angesteuert werden, dass die so produzierte Lösung für z.B. Dialysebehandlungen oder Infusionsbehandlungen den von der Blutbehandlungseinheit und der Schnittstelle zur Dateneingabe festgelegten Kriterien entspricht.

In einer bevorzugten Ausführungsform wird durch die Blutbehandlungseinheit der Massen- oder Volumenstrom vorgegeben, während durch Eingabe von Patientendaten in die Schnittstelle zur Dateneingabe die Zusammensetzung der gemischten Lösung vorgegeben wird. Die Steuer- oder Regeleinrichtung wird also die Lösungen für die Patientenbehandlung in gegebener Menge und gegebener Zusammensetzung produzieren können. Die gebrauchsfertige Lösung kann dann durch einen Fluidauslass aus der Vorrichtung entnommen werden.

In einer bevorzugten Ausführungsform mündet der Fluidauslass in die Dosiervorrichtung der Blutbehandlungseinheit, mit der bei Blutbehandlungseinheiten nach dem Stand der Technik der Fluss von der sauren Komponente (Komponente A) gesteuert wird.

In seiner bevorzugten Ausführungsform richtet sich die Anzahl der Fluideinlässe (A1, A2, A3, A4, An) nach der Anzahl der Stoffe, die in der fertig gemischten Lösung am Fluidauslass unabhängig voneinander variiert werden sollen, und werden für durch folgende Formel beschrieben: ${n}_{i} = {2}^{{n}_{s}}$ *nᵢ* Anzahl der Fluideinlässe
*nₛ* Anzahl der Stoffe, die variiert werden sollen

Möchte man beispielsweise zwei Stoffe S_1 und S_2 der Lösung für die Dialyse- oder Infusionsbehandlung innerhalb der Grenzen min und max variieren, müssen vier Lösungen (nachfolgend auch "Basisrezepturen") an vier Fluideinlässe (A1, A2, A3, A4) angeschlossen werden, in denen die zwei zu variierenden Stoffe in folgender Konzentration vorliegen können:

| Einlass *A*₁ | Einlass *A*₂ | Einlass *A*₃ | Einlass *A*₄ |
|---|---|---|---|
| *S*_{1,min} | *S*_{1,max} | *S*_{1,min} | *S*_{1,max} |
| S_{2,min} | *S*_{2,min} | *S*_{2,max} | *S*_{2,max} |
| *S*₃ | *S*₃ | *S*₃ | *S*₃ |
| *S*₄ | *S*₄ | *S*₄ | *S*₄ |
| ↓...↓ | ↓...↓ | ↓...↓ | ↓...↓ |
| *Sₙ* | *Sₙ* | *Sₙ* | *Sₙ* |

In diesem Beispiel sind an jedem Fluideinlass (A1, A2, A3, A4, An) der Mischvorrichtung gebrauchsfertige Lösungen angeschlossen, die sich nur in den zu variierenden Stoffkonzentrationen voneinander unterscheiden.

In einer bevorzugten Ausführungsform handelt es sich bei jedem der angeschlossenen Basislösungen um arzneimittelrechtlich zugelassene Lösungen.

Durch die Steuer- und Regeleinrichtung werden die Dosiervorrichtungen an den Fluideinlässen derart angesteuert, dass über das Mischungsverhältnis der Basisrezepturen zueinander, die durch die Schnittstelle zur Dateneingabe vorgegebene Stoffzusammensetzung innerhalb der Grenzen min und max am Fluidauslass vorliegt und der benötigte Volumen- oder Massenstrom eingestellt wird.

Wenn jede einzelne Basisrezeptur für sich genommen arzneimittelrechtlich zugelassen ist, kann selbst bei einer vollständigen Fehlfunktion der Vorrichtung niemals eine Stoffkonzentration außerhalb des Intervalls min und max am Fluidauslass vorliegen.

Befinden sich die Grenzen min und max in für eine Dialyse- oder Infusionsbehandlung zugelassenen Grenzen oder werden durch den Bediener der technischen Vorrichtung gemäß des individuellen Patientenrisikos ausgewählt, wird bei Fehlfunktion der technischen Vorrichtung der Patient zwar schlechter behandelt, die Stoffkonzentrationen in der gemischten Lösung am Fluidauslass können jedoch niemals einen patientengefährdenen Bereich erreichen.

Dies ist anders als in der durch DE102013102914A1 offenbarten technischen Vorrichtung, bei der hochkonzentrierte Stoffe außerhalb der bestehenden Zulassungsgrenzen direkt in eine Reinwasserleitung gefördert werden, wodurch auch zu hohe oder niedrige Konzentrationen möglich sind.

Alle anderen Stoffe *S*_{3...*n*} in den angeschlossenen Basisrezepturen sind vorzugsweise an jedem Fluideinlass (A1, A2, A3, A4) in gleicher Stoffmenge vorhanden, und ändern daher am Fluidauslass der technischen Vorrichtung ihre Stoffkonzentration in der gemischten Lösung nicht.

Am Beispiel einer Dialysebehandlung können als zu variierenden Stoffe Kalium- und Calciumionen gewählt werden, während Natrium-, Magnesium-, Acetationen und Glucose auf diesem Weg nicht variieren. Je nach Therapie kann aber jeder Stoff für eine Variation vorgesehen werden. Es können gemäß. o.g. Formel auch mehr als zwei Stoffe variiert werden, genauso kann nur ein Stoff variiert werden.

Bei kationischen Stoffen ist zu erwähnen, dass die anionischen Stoffe (zumeist Chloridionen) entsprechend der elektrischen Neutralität mitverändert werden, allerdings nicht in den Formeln zur Ermittlung der Anzahl der Fluideinlässe berücksichtigt werden.

Möchte man beispielsweise die Konzentration der Kaliumionen in der fertig gemischten Lösung variieren, werden die Chloridionen im gleichen Maße mitvariiert, da das Kalium in Lösungen für die Dialyse zumeist in Form von gelöstem Kaliumchlorid vorliegt. Dennoch wählt man *nₛ* = 1 , auch wenn mit Kalium und Chlorid zwei Stoffe variiert werden, doch Kalium und Chlorid können nicht unabhängig voneinander variiert werden.

In einer anderen Ausführungsform kann das Flüssigkeitsleitungssystem als Schlauchsystem ausgeführt sein, welches für den einmaligen Gebrauch bestimmt ist. Diese Ausführungsform kann vor allem für den Einsatz in Kleinserien wie z.B. für die klinische Erprobung der Anlage bevorzugt werden, da ein solches Schlauchsystem als Einwegartikel gut sterilisierbar ist und somit die Hygiene sichergestellt werden kann. Die Dosiervorrichtungen könnten in so einem Fall als peristaltische Schlauchpumpen ausgeführt werden.

### Beschreibung der Figuren

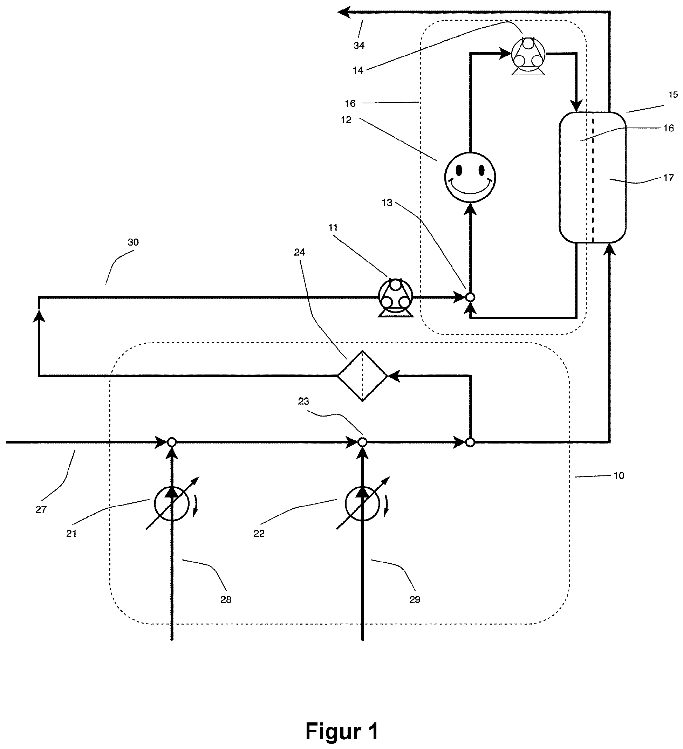

- Figur 1: Schematische und exemplarische Darstellung der Hämodiafiltration gemäß dem Stand der Technik
- Figur 2: Schematische und exemplarische Darstellung der Hämodiafiltration unter Einsatz einer erfindungsgemäßen Ausführungsform der Erfindung, wobei die Vorrichtung so ausgestaltet ist, dass sie in die Blutbehandlungseinheit integriert ist

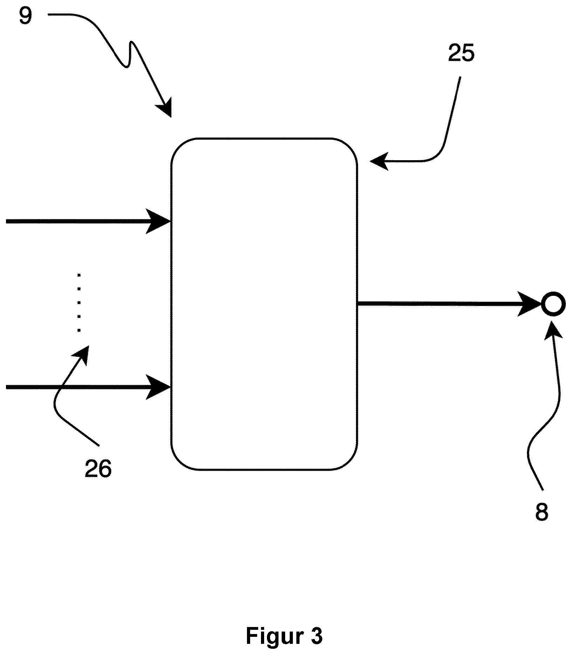

- Figur 3: Exemplarische Ausführung der erfindungsgemäßen Mischvorrichtung

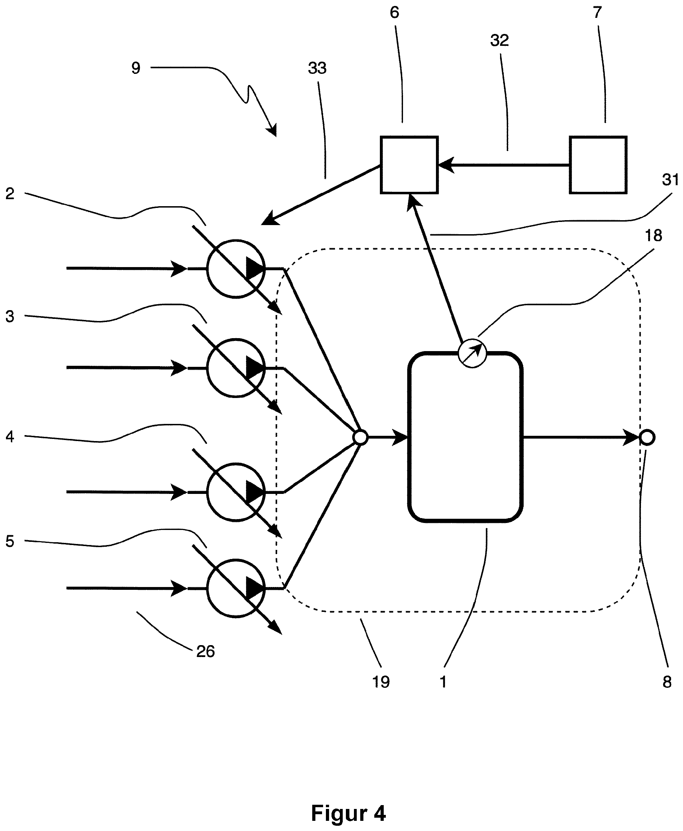

- Figur 4: Vorteilhafte Ausführungsform der erfindungsgemäßen Vorrichtung, wobei vier Fluideinlässe zur unabhängigen Variation von zwei Stoffen vorliegen und das gemeinsame Flüssigkeitsleitungssystem ein Reservoir mit Sensoreinrichtung umfasst

**Nachfolgend werden bevorzugte Aspekte der Offenbarung beschrieben.**
1. Technische Mischvorrichtung zur patientenindividuellen Herstellung von gebrauchsfertigen oder konzentrierten Lösungen für Dialyse- oder Infusionsbehandlungen, wobei durch den Aufbau der Vorrichtung bestimmte Konzentrationsgrenzen bestimmter Stoffe inder Zusammensetzung der fertig gemischten Lösung nicht unter- oder überschritten werden können, während die Stoffzusammensetzung der fertig gemischten Lösung vor und während der Behandlung variiert werden kann, mit:
   - mindestens zwei Fluideinlässen (26) , an welche ausschließlich Basislösungen angeschlossen werden, die sich ihrerseits innerhalb der Konzentrationsgrenzen befinden und sich nur in den Stoffmengen der am Fluidauslass (8) zu variierenden Stoffe voneinander unterscheiden;
   - einer Mischeinrichtung (25), welche die an den Fluideinlässen angeschlossenen Basislösungen derart quantitativ mischt, dass die gemischte Lösung am Fluidauslass (8) den benötigten Volumen- oder Massenstrom aufweist und in seiner Stoffzusammensetzung der benötigten Stoffzusammensetzung entspricht;
   - mindestens einem Fluidauslass (8), an dem sich die Stoffzusammensetzung der fertig gemischten Lösung innerhalb der durch die an den Fluideinlässen (26) angeschlossenen Basislösungen definierten Zusammensetzungsintervallen befindet, wobei sich diese Intervalle, da ausschließlich Basislösungen vermischt werden, deren Stoffzusammensetzungen sich ihrerseits innerhalb bestimmter Konzentrationsgrenzen befinden, auch nur innerhalb dieser Konzentrationsgrenzen befinden können.
2. Technische Mischvorrichtung wie oben unter Punkt 1 beschrieben, wobei sie durch folgende Bauteile ausgebildet ist:
   - einer Anzahl ${n}_{i} = {2}^{{n}_{s}}$
   - Fluideinlässe (26) zur voneinander unabhängigen Variation einer Anzahl *nₛ* Inhaltsstoffe in der fertig gemischten Lösung für Dialyse- oder Infusionsbehandlungen, an welche ausschließlich Basislösungen angeschlossen werden, die sich ihrerseits innerhalb rechtlicher (z.B. Standardzulassung) oder therapeutischer Grenzen befinden und sich nur in den Stoffmengen der am Fluidauslass (8) zu variierenden Stoffe voneinander unterscheiden;
   - je Fluideinlass (26) jeweils mindestens einer Dosiervorrichtung (2,3,4,5), die in fluidischer Verbindung mit einem gemeinsamen Flüssigkeitsleitungssystem (19) stehen;
   - mindestens einem gemeinsamen Flüssigkeitsleitungssystem (19), welches ein Flüssigkeitsreservoir (1) umfassen kann, und mindestens eine Konvenktioniereinrichtung enthalten kann;
   - mindestens einer Schnittstelle zur Dateneingabe (7), die Daten vom Benutzer, Patientendaten oder Daten von der Blutbehandlungseinheit (10) empfangen und der Vorrichtung (9), und insbesondere einer Steuer- oder Regeleinrichtung (6), zur Verfügung stellen kann;
   - mindestens einer Steuer- oder Regeleinrichtung (6), die die Dosiervorrichtungen der Fluideinlässe derart ansteuert oder einregelt, dass jede am Fluideinlass (26) angeschlossene Basislösung dem gemeinsamen Flüssigkeitsleitungssystem (19) quantitativ zugemischt wird, sodass die Lösung für Dialyse- oder Infusionsbehandlungen den am Fluidauslass (8) benötigten Volumen- oder Massenstrom aufweist und in seiner Stoffzusammensetzung der durch die Schnittstelle zur Dateneingabe (7) definierten Stoffzusammensetzung entspricht;
   - mindestens einem Fluidauslass (8) für die fertig gemischte Lösung für die Dialyse- oder Infusionsbehandlung, welcher ebenfalls eine Dosiervorrichtung umfassen kann.
3. Technische Mischvorrichtung (9), die ergänzend zu den Angaben unter Punkt 2 oben dadurch beschrieben wird, dass sie mindestens eine Sensoreinrichtung (18) umfasst, die in der erfindungsgemäßen Mischvorrichtung (9) mindestens eine physikalische Größe misst und dieses Messergebnis der Steuer- oder Regeleinrichtung (6) zur Verfügung stellt.
4. Technische Mischvorrichtung (9), wie oben unter den Ziffern 1, 2 oder 3 beschrieben, die weiter dadurch konkretisiert ist, dass sie als integraler Bestandteil einer Blutbehandlungseinheit (10) ausgebildet ist.
5. Technische Mischvorrichtung (9) wie oben unter den Ziffern 1, 2 oder 3 beschrieben, die weiter dadurch gekennzeichnet ist, dass sie sich außerhalb einer Blutbehandlungseinheit befindet und an diese konnektiert wird.
6. Technische Mischvorrichtung (9) wie sie in einem der obigen Absätze beschrieben ist, bei der das gemeinsame Flüsssigkeitsleitungssystem (19) ein Reservoir (1) enthält.
7. Technische Mischvorrichtung (9) wie sie in einem der vorherigen Absätze 1 bis 6 beschrieben ist, wobei die Zahl der Eingänge auf 4 Eingänge zur voneinander unabhängigen Variation der Konzentration von 2 Stoffen am Fluidauslass (8) der technischen Mischvorrichtung (9) festgelegt wird.
8. Technische Mischvorrichtung (9) nach einem der Absätze 1 bis 6, bei der die Zahl der Eingänge auf 2 Eingänge zur voneinander unabhängigen Variation der Konzentration von einem Stoff am Fluidauslass (8) der technischen Mischvorrichtung (9) festgelegt wird.
9. Technische Mischvorrichtung (9) wie oben in den Absätzen beschrieben, in der die Dosiervorrichtung (26) als Pumpen oder Ventile ausgestaltet sind.
10. Technische Mischvorrichtung (9) wie oben in den Absätzen beschrieben, wobei die Steuer- oder Regeleinheit (6) als programmierbarer Prozessor ausgebildet ist.

### Bezugszeichenliste

- 1: Reservoir in der erfindungsgemäßen Mischvorrichtung
- 2: Dosiervorrichtung an Einlass A₁
- 3: Dosiervorrichtung an Einlass A₂
- 4: Dosiervorrichtung an Einlass A₃
- 5: Dosiervorrichtung an Einlass A₄
- 6: Steuer- oder Regeleinheit
- 7: Schnittstelle zur Dateneingabe
- 8: Fluidauslass
- 9: Erfindungsgemäße technische Mischvorrichtung
- 10: Die Blutbehandlungseinheit bzw. seine Systemgrenze
- 11: Peristaltische Substitutionslösungspumpe
- 12: Patient
- 13: Infusionsstelle im extrakorporalen Blutkreislauf (in allen Figuren Postdilution)
- 14: Peristaltische Blutpumpe
- 15: Dialysefilter
- 16: Blutkammer des Dialysefilters
- 17: Spüllösungskammer des Dialysefilters
- 18: Sensoreinrichtung
- 19: Flüssigkeitsleitungssystem in der technischen Mischvorrichtung
- 20: Extrakorporaler Blutkreislauf
- 21: Flusskontrolleinrichtung für die basische Komponente (B)
- 22: Flusskontrolleinrichtung für die saure Komponente (A)
- 23: Verdünnungsstellen, bei denen jeweils die saure oder basische Komponente mit Reinwasser und der zuvor zugeführten Komponente vermischt werden
- 24: Sterilfilter
- 25: Mischeinrichtung
- 26: Einlässe Aₙ
- 27: Reinwasser
- 28: Einlass B
- 29: Einlass A
- 30: Substitutionslösung
- 31: Leitung Sensoreinrichtung
- 32: Informationsleitung Zusammensetzung
- 33: Leitung Steuersignal
- 34: Verbrauchte Dialyselösung

## Patentansprüche

1. Mischvorrichtung mit mindestens zwei Fluideinlässen (26), einer Mischeinrichtung (25) und mindestens einem Fluidauslass (8) zur Herstellung eines Dialysats oder einer Substitutionslösung zur Verabreichung am Menschen während einer extrakorporalen Blutbehandlung, **dadurch gekennzeichnet, dass** :
- an den mindestens zwei Fluideinlässen (26) Basislösungen angeschlossen werden, die innerhalb vordefinierter Konzentrationsgrenzen liegen, und die sich nur in den Stoffmengen der am Fluidauslass (8) zu variierenden Stoffe voneinander unterscheiden;
- die Mischeinrichtung (25) die an den Fluideinlässen (26) angeschlossenen Basislösungen derart quantitativ mischt, dass die gemischte Lösung am Fluidauslass (8) den benötigten Volumen- oder Massenstrom aufweist, und in seiner Stoffzusammensetzung der benötigten Stoffzusammensetzung entspricht;
- sich an dem mindestens einen Fluidauslass (8) die Stoffzusammensetzung der fertig gemischten Lösung innerhalb der durch die an den Fluideinlässen (26) angeschlossenen Basislösungen definierten Zusammensetzungsintervallen befindet.

2. Mischvorrichtung nach Anspruch 1, **dadurch gekennzeichnet, dass** die Vorrichtung durch die folgenden Elemente umfasst:
- einer Anzahl ${n}_{i} = {2}^{{n}_{s}}$ Fluideinlässe (26) zur Variation einer Anzahl *nₛ* Inhaltsstoffe in einer daraus gemischten Lösung zur Verabreichung am Menschen, die sich nur in den Stoffmengen der am Fluidauslass (8) zu variierenden Stoffe voneinander unterscheiden;
- je Fluideinlass (26) jeweils mindestens einer Dosiervorrichtung (2,3,4,5), die in fluidischer Verbindung mit einem gemeinsamen Flüssigkeitsleitungssystem (19) stehen;
- mindestens einem gemeinsamen Flüssigkeitsleitungssystem (19), welches ein Flüssigkeitsreservoir (1) umfassen kann, und mindestens eine Konvenktioniereinrichtung enthalten kann;
- mindestens einer Schnittstelle zur Dateneingabe (7), die Daten vom Benutzer, Patientendaten oder Daten von der Blutbehandlungseinheit (10) empfangen und der Vorrichtung (9), und insbesondere einer Steuer- oder Regeleinrichtung (6), zur Verfügung stellen kann;
- mindestens einer Steuer- oder Regeleinrichtung (6), die die Dosiervorrichtungen (2, 3, 4, 5) der Fluideinlässe (26) derart ansteuert oder einregelt, dass jede am Fluideinlass (26) angeschlossene Basislösung dem gemeinsamen Flüssigkeitsleitungssystem (19) quantitativ zugemischt wird, sodass die Lösung für Dialyse- oder Infusionsbehandlungen den am Fluidauslass (8) benötigten Volumen- oder Massenstrom aufweist und in seiner Stoffzusammensetzung der durch die Schnittstelle zur Dateneingabe (7) definierten Stoffzusammensetzung entspricht;
- mindestens einem Fluidauslass (8) für die fertig gemischte Lösung für die Verabreichung am Menschen, welcher ebenfalls eine Dosiervorrichtung umfassen kann.

3. Mischvorrichtung gemäß Anspruch 2, **dadurch gekennzeichnet, dass** sie mindestens eine Sensoreinrichtung (18) umfasst, die mindestens eine physikalische Größe misst und dieses Messergebnis der Steuer- oder Regeleinrichtung (6) zur Verfügung stellt.

4. Mischvorrichtung (9) gemäß einem der Ansprüche 1-3, **dadurch gekennzeichnet, dass** sie als integraler Bestandteil einer Blutbehandlungseinheit (10) ausgebildet ist.

5. Mischvorrichtung (9) gemäß einem der Ansprüche 1-3, **dadurch gekennzeichnet, dass** sie sich außerhalb einer Blutbehandlungseinheit befindet und an diese konnektiert wird.

6. Mischvorrichtung (9) gemäß einem der vorhergehenden Ansprüche, **dadurch gekennzeichnet, dass** das gemeinsame Flüsssigkeitsleitungssystem (19) ein Reservoir (1) enthält.

7. Mischvorrichtung (9) gemäß einem der Ansprüche 1-6, **dadurch gekennzeichnet, dass** die Zahl der Eingänge auf vier Eingänge zur voneinander unabhängigen Variation der Konzentration von zwei Stoffen am Fluidauslass (8) der Mischvorrichtung (9) festgelegt wird.

8. Mischvorrichtung (9) gemäß einem der Ansprüche 1-6, **dadurch gekennzeichnet, dass** die Zahl der Eingänge auf zwei Eingänge zur voneinander unabhängigen Variation der Konzentration von einem Stoff am Fluidauslass (8) der Mischvorrichtung (9) festgelegt wird.

9. Mischvorrichtung (9) gemäß einem der vorhergehenden Ansprüche, **dadurch gekennzeichnet, dass** die Dosiervorrichtung (26) als Pumpe oder Ventil ausgestaltet ist.

10. Mischvorrichtung (9) gemäß einem der vorhergehenden Ansprüche, **dadurch gekennzeichnet, dass** die Steuer- oder Regeleinheit (6) als programmierbarer Prozessor ausgebildet ist.

## Claims

1. Mixing device with at least two fluid inlets (26), a mixing facility (25) and at least one fluid outlet (8) for the production of a dialysate or a substitution solution for administration to humans during extracorporeal blood treatment, **characterised in that**:
- basic solutions are connected to the at least two fluid inlets (26), which are within predefined concentration limits and which differ from one another only in the substance quantities of the substances to be varied at the fluid outlet (8);
- the mixing facility (25) quantitatively mixes the basic solutions connected to the fluid inlets (26), such that the mixed solution has the required volume or mass flow at the fluid outlet (8) and corresponds in its substance composition to the required substance composition;
- at the at least one fluid outlet (8), the substance composition of the ready-mixed solution is within the composition intervals defined by the basic solutions connected to the fluid inlets (26).

2. Mixing device according to claim 1, **characterised in that** the device comprises the following elements:
- a number ${n}_{i} = {2}^{{n}_{s}}$ of fluid inlets (26) for varying a number of *nₛ* of ingredients in a solution mixed therefrom for administration to humans, which differ from one another only in the substance quantities of the substances to be varied at the fluid outlet (8);
- for each fluid inlet (26), at least one dosing device (2,3,4,5) in each case, which are in fluidic connection with a common liquid line system (19);
- at least one common liquid line system (19), which may comprise a liquid reservoir (1), and may contain at least one convection facility;
- at least one interface for data input (7), which can receive data from the user, patient data or data from the blood treatment unit (10) and make it available to the device (9), and in particular to a control or regulating facility (6);
- at least one control or regulating facility (6), which controls or regulates the dosing devices (2, 3, 4, 5) of the fluid inlets (26), such that each basic solution connected to the fluid inlet (26) is quantitatively admixed with the common liquid line system (19), such that the solution for dialysis or infusion treatments has the volume or mass flow required at the fluid outlet (8) and corresponds in its substance composition to the substance composition defined by the interface for data input (7);
- at least one fluid outlet (8) for the ready-mixed solution for administration to humans, which may also comprise a dosing device.

3. Mixing device according to claim 2, **characterised in that** it comprises at least one sensor facility (18) which measures at least one physical variable and makes this measurement result available to the control or regulating facility (6).

4. Mixing device (9) according to any of claims 1-3, **characterised in that** it is designed as an integral component of a blood treatment unit (10).

5. Mixing device (9) according to any of claims 1-3, **characterised in that** it is located outside and connected to a blood treatment unit.

6. Mixing device (9) according to any of the preceding claims, **characterised in that** the common liquid line system (19) contains a reservoir (1).

7. Mixing device (9) according to any of claims 1-6, **characterised in that** the number of inputs is set to four inputs for independently varying the concentration of two substances at the fluid outlet (8) of the mixing device (9).

8. Mixing device (9) according to any of claims 1-6, **characterised in that** the number of inputs is set to two inputs for independently varying the concentration of a substance at the fluid outlet (8) of the mixing device (9).

9. Mixing device (9) according to any of the preceding claims, **characterised in that** the dosing device (26) is designed as a pump or valve.

10. Mixing device (9) according to any of the preceding claims, **characterised in that** the control or regulation unit (6) is designed as a programmable processor.

## Revendications

1. Dispositif mélangeur doté d'au moins deux entrées de fluide (26), d'un moyen de mélange (25) et d'au moins une sortie de fluide (8) pour la préparation d'un dialysat ou d'une solution de substitution permettant l'administration à l'homme pendant un traitement extracorporel du sang, **caractérisé en ce que** :
- sur lesdites au moins deux entrées de fluide (26), des solutions de base qui se trouvent dans des limites de concentration prédéfinies et qui ne diffèrent les unes des autres que par les quantités de substances devant être modifiées au niveau de la sortie de fluide (8) ;
- le moyen de mélange (25) mélange de manière quantitative les solutions de base raccordées aux entrées de fluide (26) de sorte que la solution mélangée présente le débit volumique ou massique requis à la sortie de fluide (8) et corresponde dans sa composition à la composition de substance requise ;
- au niveau de ladite au moins une sortie de fluide (8), la composition de la solution mélangée prête à l'emploi se trouve à l'intérieur des intervalles de composition définis par les solutions de base raccordées aux entrées de fluide (26).

2. Dispositif mélangeur selon la revendication 1, **caractérisé en ce que** le dispositif comprend les éléments suivants :
- un nombre ${n}_{i} = {2}^{{n}_{s}}$ d'entrées de fluide (26) pour faire varier un nombre *nₛ* d'ingrédients dans une solution mélangée pour l'administration à l'homme, qui ne diffèrent les uns des autres que par les quantités de substances devant être modifiées au niveau de la sortie de fluide (8) ;
- par entrée de fluide (26), au moins un dispositif de dosage (2,3,4,5), qui est en communication fluidique avec un système de conduite de fluide commun (19) ;
- au moins un système de conduite de fluide commun (19), qui peut comprendre un réservoir de liquide (1), et peut comprendre au moins un moyen de convection ;
- au moins une interface pour la saisie des données (7), qui peut recevoir des données de l'utilisateur, des données du patient ou des données de l'unité de traitement du sang (10) et les mettre à la disposition du dispositif (9), et en particulier d'un dispositif de commande ou de régulation (6) ;
- au moins un dispositif de commande ou de régulation (6) qui commande ou régule les dispositifs de dosage (2, 3, 4, 5) de l'entrée de fluide (26) de sorte que chaque solution de base raccordée à l'entrée de fluide (26) soit mélangée de manière quantitative au système de conduite de fluide commun (19), de sorte que la solution pour les traitements de dialyse ou de perfusion présente le débit volumique ou massique requis à la sortie de fluide (8) et corresponde dans sa composition à la composition de substance définie par l'interface de saisie de données (7) ;
- au moins une sortie de fluide (8) pour la solution prête à l'emploi permettant l'administration à l'homme, qui peut également comprendre un dispositif de dosage.

3. Dispositif mélangeur selon la revendication 2, **caractérisé en ce qu'**il comprend au moins un dispositif de détection (18) qui mesure au moins une grandeur physique et met ce résultat de mesure à la disposition du dispositif de commande ou de régulation (6).

4. Dispositif mélangeur (9) selon l'une quelconque des revendications 1 à 3, **caractérisé en ce qu'**il est réalisé en tant que partie intégrante d'une unité de traitement du sang (10).

5. Dispositif mélangeur (9) selon l'une quelconque des revendications 1 à 3, **caractérisé en ce qu'**il se trouve à l'extérieur d'une unité de traitement du sang et est raccordé à celle-ci.

6. Dispositif mélangeur (9) selon l'une quelconque des revendications précédentes, **caractérisé en ce que** le système de conduite de fluide commun (19) comprend un réservoir (1).

7. Dispositif mélangeur (9) selon l'une quelconque des revendications 1 à 6, **caractérisé en ce que** le nombre d'entrées est fixé à quatre entrées pour faire varier indépendamment l'une de l'autre les concentrations de deux substances au niveau de la sortie de fluide (8) du dispositif mélangeur (9).

8. Dispositif mélangeur (9) selon l'une quelconque des revendications 1 à 6, **caractérisé en ce que** le nombre d'entrées est fixé à deux entrées pour faire varier indépendamment la concentration d'une sortie de fluide (8) du dispositif mélangeur (9).

9. Dispositif mélangeur (9) selon l'une quelconque des revendications précédentes, **caractérisé en ce que** le dispositif de dosage (26) est conçu comme une pompe ou une soupape.

10. Dispositif mélangeur (9) selon l'une quelconque des revendications précédentes, **caractérisé en ce que** l'unité de commande ou de régulation (6) est réalisée sous la forme d'un processeur programmable.
